# EUROPEAN PATENT APPLICATION

(11) **EP 3 363 778 A1**
(43) Date of publication of application: **22.08.2018**
(21) Application number: 17156583.1
(22) Date of filing: 17.02.2017
(51) Int. Cl.: C07C 51/285, C07C 63/26

(54) **PROCESS FOR THE OXIDATION OF ALKYLAROMATIC COMPOUNDS**

(71) Applicant: Solvay SA, 1120 Brussels (BE)
(72) Inventor: DOURNEL, Pierre, 1030 BRUSSELS (BE); DESMEDT, Frédérique, 1050 Brussels (BE); MIQUEL, Pierre, deceased (FR); VLASSELAER, Yves, 3061 LEEFDAAL (BE)

(57) **Abstract**

Process for oxidising an alkylaromatic compound, said process comprising a step of reacting the alkylaromatic compound with hydrogen peroxide in the presence of a catalyst comprising iron perchlorate or iron tetrafluoroborate.

## Description

This invention concerns a process for the oxidation of alkylaromatic compounds using an oxidising system comprising an iron salt and hydrogen peroxide. In one embodiment, the invention concerns the oxidation of p-xylene in TerePhthalic Acid (TPA) using such a system.

TPA is a major chemical building block used today mainly to produce PET (polyethylene terephthalate), a common thermoplastic polymer.

Today the industrial process for producing terephthalic acid is mainly based on the so-called Amoco Process. This process is based on an air oxidation at high temperature (175 - 225°C) between 15 and 30 bars and in presence of acetic acid as solvent (used to solubilize the TPA and the p-toluic acid (main intermediate product)). The catalytic system is a combination of cobalt, manganese and bromide ions. The preferred system is composed of cobalt acetate, manganese acetate and hydrobromic acid. The bromides play a key role in the system. The bromine form initiates the hydrogen abstraction from the reactant (p-xylene) giving the bromide which helps to loop the redox system Co2+ / Co3+ -Mn2+ / Mn3+ and, by this way, allows the recycling of the catalytic system.

High yield in terephthalic acid and high selectivity are obtained in this process. However, some major drawbacks exist as the high working temperature needed to activate the air oxidation and the use of bromides and concentrated acetic acid which are an important source of corrosion.

In the past, alkylaromatic oxidation processes using hydrogen peroxide as oxidant have also been described which are working at lower temperature.

US 5,473,101 for instance discloses a process for the controlled oxidation of alkylaromatic compounds comprises reacting said compounds with an oxidising system comprising cobalt (II) ions, bromide ions, and hydrogen peroxide, in the presence of an appropriate protic solvent. The process is particularly useful in the selective oxidation of poly(alkyl)aromatic compounds. However, as appears namely from Example 11 thereof in which p-xylene is oxidised, this process only yields products like tolualdehyde and toluic acid and no polyoxidised products such as TPA.

More recent attempts to use hydrogen peroxide as oxidant to make TPA starting from p-xylene have been made but they make often use of supercritical conditions which imply complicated synthesis processes.

The present invention is based on the surprising finding that simple catalysts to which hydrogen peroxide is normally sensitive to decomposition, give excellent results in oxidising alkylaromatics in acids and more precisely, in oxidising p-xylene to TPA using hydrogen peroxide even at low temperature, without bromide addition and in the presence of a solvent which does not need to be pure or concentrated acetic acid.

Therefore, the present invention relates to a process for oxidising an alkylaromatic compound, said process comprising a step of reacting the alkylaromatic compound with hydrogen peroxide in the presence of a catalyst comprising iron perchlorate or iron tetrafluoroborate.

The alkylaromatic compounds which are oxidised most satisfactorily by the process of the present invention are those which comprise at least one alkyl substituent, preferably a (C1-C6) alkyl substituent having at least one hydrogen atom at the alpha position relative to the aromatic ring. Such hydrogen atoms are sometimes called benzilic hydrogens. From the preferred group of substituents, straight chain (C1-C6) alkyl substituents and branched chain alkyl substituents having less than 5 carbons are most preferred. More preferably, the substituent is a C1 or C2 alkyl substituent, most preferably a C1 alkyl substituent. Typical alkylaromatic compounds which may be oxidised by the present invention include mono-, di- or tri-alkylbenzenes, such as toluene, ethylbenzene, p-t-butyltoluene, cumene, o-, m- or p-xylenes, o-, m-, p-diethylbenzenes, tri-methylbenzenes, e.g. mesitylene, and polynuclear alkylaromatic compounds such as the mono-, di- and tri-alkyl naphthalenes, e.g. methyl naphthalenes, ethyl naphthalenes and dimethylnaphthalenes. Preferably, it is a mononuclear alkylaromatic compound and preferably, a di-substituted one. The invention gives good results with xylene and more particularly, with p-xylene.

Generally, from 0.1 to 6 moles, preferably, from about 0.2 to about 1.5 moles, more preferably from about 0.4 to about 1.1 moles of hydrogen peroxide are present per mole of alkylaromatic compound to be oxidised. The hydrogen peroxide is preferably added as an aqueous solution, which solution desirably has a strength of at least 25 percent peroxide. Preferably the solution is not stronger than 85 percent peroxide. The preferred solution has a strength of from about 35 percent to about 70 percent peroxide. In practice, crude hydrogen peroxide from an AO process can be used (i.e. a solution coming directly from the process without further concentration and/or purification). Therefore, in a preferred embodiment, the hydrogen peroxide is an aqueous solution having a maximum concentration between 40 and 55wt %, more preferably between 40 and 44 % (more concentrated solutions requiring a distillation step). The reaction of the invention generally leads to the formation of 2 or 3 phases : a liquid organic phase, an aqueous phase and a solid phase if some reaction products and/or by products are at least partly insoluble in both liquid phases.

In the case of oxidation of p-xylene to TPA, the more concentrated the hydrogen peroxide solution used, the more solid is obtained which solid comprising most of the TPA and the toluic acid, the rest of these compounds being in the organic phase together with the organic reaction by-products and other intermediaries.

The reaction can be performed directly in the alkylaromatic compound; however, preferably, it is performed in presence of an organic solvent like acetonitrile (AcN) or a mixture of acetonitrile and acetic acid (AcAc) which gives good results for the p-xylene oxidation in TPA, the acetic acid helping to dissolve the TPA and its intermediate compounds like p-toluic acid. Preferably, acetonitrile comprising a minor amount of acetic acid, for instance from 1 to 50wt %, preferably from 2 to 30wt % and even more preferably from 5 to 15wt % in weight, is used. It is namely so that the oxidation of p-xylene is a reaction in which said p-xylene is successively oxidised as follows :
p-xylene to 4-methyl- benzyl alcohol to p-tolualdehyde and then either :
   A. to p-toluic acid to 4-hydroxy- benzoic acid to 4-formylbenzoic acid to terephthalic acid
   B. to 4-hydroxybenzaldehyde to terephtaldehyde to 4-formylbenzoic acid to terephthalic acid.

Acetic acid helps to solubilize all these compounds hence helping the reaction to proceed to the final compound (TPA) by preventing intermediate species (like toluic acid) to precipitate out of the reaction medium.

Other possible candidates are for instance diisobutyl carbinol (DBC), N-octanol, ethyl acetate or dimethyl formamide (DMF) either pure or in mixture with AcAc, especially again for the oxidation of p-xylene to TPA and this for the same reason (dissolution of intermediates and by-products, namely toluic acid).

Generally, the reaction is conducted in batch and after the reaction is completed, the reaction products are preferably isolated from the solvent and/or the catalyst. In the oxidation of p-xylene to TPA, this can for instance be done by a process comprising at least one of the following steps :
1. Filtration and solid phase recovering
2. Solubilisation of the solid in an adequate solvent and eventually recrystallization to recover TPA
3. Separation between organic & aqueous phase
4. Distillation of the organic phase for solvent recovering
5. Treatment of the aqueous phase for catalyst recovering (for instance by ion exchange on resins).

In practice, it is convenient to fill a reactor with the catalyst, the alkylaromatic compound and the solvent, the case being, and to circulate a flow of hydrogen peroxide through this reactor until reaching the required quantity of hydrogen peroxide (see above).

In a preferred embodiment, the reaction is performed in presence of oxygen. More preferably, a gas flow comprising oxygen (for instance air or a mixture of oxygen and nitrogen) is introduced in the reaction medium, for instance by circulating such a flow through a reactor as set forth above. The gas flow is preferably started together with the hydrogen peroxide introduction. Preferably, the gas flow is pursued after the hydrogen peroxide addition has been completed. This embodiment gives especially good results in the oxidation of p-xylene to TPA.

The flows of hydrogen peroxide and of oxygen, the case being, are preferably adapted in order to have an optimal hydrogen peroxide efficiency and an optimal conversion of the alkylaromatic compound, and so are the other reaction conditions by the way.

For instance, the reaction pressure is generally of at least 1 bara and up to 100 bara, preferably however below 80 bara and even more preferably below 50 bara.

The reaction temperature is generally from 0 to 150°C, preferably from 10 to 120°C, even more preferably from 20 to 100°C.

The reaction time usually is in the range of hours, for instance from 0.25 to 24 h, preferably from 0.5 to 12 h, even more preferably from 1 to 8 h.

The ranges given above are particularly advantageous for the oxidation of p-xylene to TPA.

The process of the invention uses a catalyst. By "catalyst" is meant a substance that speeds up the chemical reaction (oxidation), but is not consumed by the reaction; hence it is recovered chemically unchanged at the end of the reaction. Typically, the catalyst is used in a molar amount which is much less than the molar amount of hydrogen peroxide and alkylaromatic compound used. Typically, the molar ratio between catalyst and hydrogen peroxide is generally below 1000, preferably below 500, more preferably below 200.

The catalyst used in the invention is iron perchlorate or iron tetrafluoroborate. It is preferably iron II or iron III perchlorate. Iron II perchlorate is the most preferred especially in the case of p-xylene oxidation.

Eventually, the iron salt catalyst is used in presence of a co-catalyst which is an organic compound like (2,6-bis-[1-(benzylimino)ethyl]pyridine - dapb). The positive effect of this compound is described in an article of Reedijk and co-workers (Journal of Molecular Catalysis A: Chemical 286 (2008), 1 - 5) in relation with the oxidation of cyclohexane, which is however much easier that the oxidation of alkylaromatic compounds. This compound (dapb) can easily be synthesized as described in this article. Generally, the catalyst /dapb molar ratio is in the range 1 to 50, preferably in the range 2 to 20 and even more preferably in the range 3 to 10. Other Schiff base tridendate complexes like dapab, salen etc. may also be used and in the same amounts.

In a preferred embodiment of the present invention, an additive is present during the reaction. As will be shown in the Examples hereafter, the Applicant has found that adding Gallic Acid or specific anthraquinones to the reaction medium can improve the TPA yield. For each of these additives, there may be an optimum concentration that can easily be determined experimentally.

Therefore, in one embodiment, the invention also relates to a process as described above wherein Gallic Acid is added before or during the reaction.

In another embodiment, the invention also relates to a process as described above wherein specific anthraquinones are added before or during the reaction. More precisely, said anthraquinones are unsubstituted or at least not substituted with hydroxyl or other oxygen containing groups. They may be substituted with aliphatic groups though and in that regard, good results have been obtained with ethylanthraquinone (EQ).

In a more preferred embodiment of the invention, the above mentioned co-catalyst is combined with the above mentioned additive. A combination of dapb and EQ gives good results as will be shown in the Examples hereafter.

According to the invention, the alkylaromatic compound is oxidized generally generating at least one acid. If the alkylaromatic compound comprises benzylic hydrogens i.e. hydrogens on a carbon atom attached to an aromatic ring, the acid function resulting from the oxidation will be located on said carbon atom. In the case of p-xylene for instance, the acids produced are listed above. It is worth noting additionally that route A is privileged and that the main acids produced are p-toluic acid and TPA.

Especially if the final goal is to produce PET from TPA, it is preferable not to have toluic acid because this compound causes problems in polymerization (it leads to short polymer chains because once this monomer is incorporated in the chain, the reaction is blocked owed to the absence of the second COOH function). Therefore, in a preferred embodiment, the invention concerns a process comprising the following steps :
- a first step in which p-xylene is oxidized with H2O2 at least partly in toluic acid and terephatic acid using a process as described above;
- a second step in which the solid produced is recovered;
- a third step in which said solid is dissolved in an appropriate solvent (for instance chosen between AcN/AcAc, DBC, N-octanol, ethyl acetate, DMF or mixture of thereof); and
- a fourth step in which the solution is oxidized preferably using a process as described above.

The invention is illustrated by the Examples below which relate to some preferred embodiments thereof and are not construed to limit its scope in any way.

### 1. Experimental procedure that was followed (written as instructions to be followed)

- Introduce in the reactor :
   ∘ the catalyst
   ∘ the p-xylene
   ∘ the solvent(s) & the co-catalyst, the case being
- Close the reactor and purge it at atmospheric pressure during some minutes under nitrogen (450 Nml/min)
- Increase the pressure to 30 bar with nitrogen (same flow)
- Strip all the oxygen from the reactor by flushing it with nitrogen (same flow). Check the oxygen vanishing by gas chromatography (on line).
- Start to heat the reactor at 90°C.
- When the temperature is reached, start the reaction : introduce in the reactor a gas mixture of nitrogen (450 Nml/min) and oxygen (590 Nml/min). Start at the same time to introduce in the reactor hydrogen peroxide with the required strength and at the required flowrate in a total amount of 50g.
- After the hydrogen peroxide addition, maintain the gases flow during 2 h 20 (total test duration = 240 minutes).
- Stop the oxygen flow and strip at 90°C the reactor during several minutes (check the oxygen content in the gas phase by GC).
- Cool down the reactor under nitrogen to 15°C.
- Stop the nitrogen flow.
- Decrease the reactor pressure to atmospheric pressure.
- Open the reactor.
- Filter the liquid phase and dry the solid (70°C)
- Separate the different liquid phases.
- Analyze the organic phase by GC
- Analyze the solid by NMR
- Analyze the aqueous phase by HPLC

### NMR analysis

A Bruker Avance equipment with a frequency of 400 MHz or 500 MHz depending on the samples, and a mixture of CD3OD and deuterated DMSO to solubilize the samples was used with octamethylcyclotetracyloxane (OMCTS) as internal standard. Confidence interval at 95 % probability of an individual measure should be about 3 %.

### Aqueous phase analysis (HPLC)

The following conditions were used :
- Column : Poroshell 120 SB-C18, 2.7 µm, 100 x 3.0 mm (Agilent); with the following pre-column : InfinityLab Poroshell 120 SB-C18, 5 x 3.0 mm, 2.7 µm (Agilent);
- Temperature : 40°C;
- Flowrate : 0.6 ml/min of a solution of methanol and acidified water (0.1 % TFA) with a gradient evolving from 10:90 to 90:10
- UV detection

### Gas phase analysis (GC)

Oxygen and nitrogen in gas phase are monitored using a Shimadzu GC-14B, fitted with a MS5A column and a TCD (Thermal Conductivity Detector) detector, in isothermal (70°C) conditions. Analyses are performed every 20 minutes.

### Organic phase analysis (GC)

### Front Inlet :

280°C
25 ml/min He
Split 100

### Column :

DB-1
60m length
320µm diameter
1µm film thickness

### Oven :

50°C - 5 min.
10°C/min
270°C - 5 min.

### Detector :

FID - 300°C
Air: 400ml/min.
Make-up : 25ml/min. (He)
H2 : 30ml/min.

Aldehydes and acids are analyzed by derivatization with BSTFA (N,O-Bis(trimethylsilyl)trifluoroacetamide)). The other products are analyzed without being derivatized.

### Calculations

1. H₂O₂ efficiency, % = N mol H₂O₂ used for intermediaries & TPA formation / N mol H₂O₂ introduced. This represents the % of hydrogen peroxide useful for the oxidation reaction. The rest is H2O2 decomposition or by-products production (not intermediaries). It is so a "hydrogen peroxide selectivity" which is an important figure from the economical point of view and for the viability of the process.
2. p-xylene Conversion, % = 1 - (N mol p-xylene residual/N mol p-xylene introduced)
3. Toluic acid Selectivity, % = N mol toluic acid formed / ∑ N mol intermediaries & TPA formed
4. TPA Selectivity, % = N mol TPA formed / ∑ N mol intermediaries & TPA formed
5. Toluic acid Yield, % = Toluic acid Selectivity * p-xylene Conversion
6. TPA Yield, % = TPA Selectivity * p-xylene Conversion

In the above, the intermediaries are all chemical compounds listed in routes A and B above, except p-xylene and TPA, and which are dosed in the solid phase, organic phase and aqueous phase.

### 2. Results obtained

The results obtained are listed in Tables 1 to 4 below.

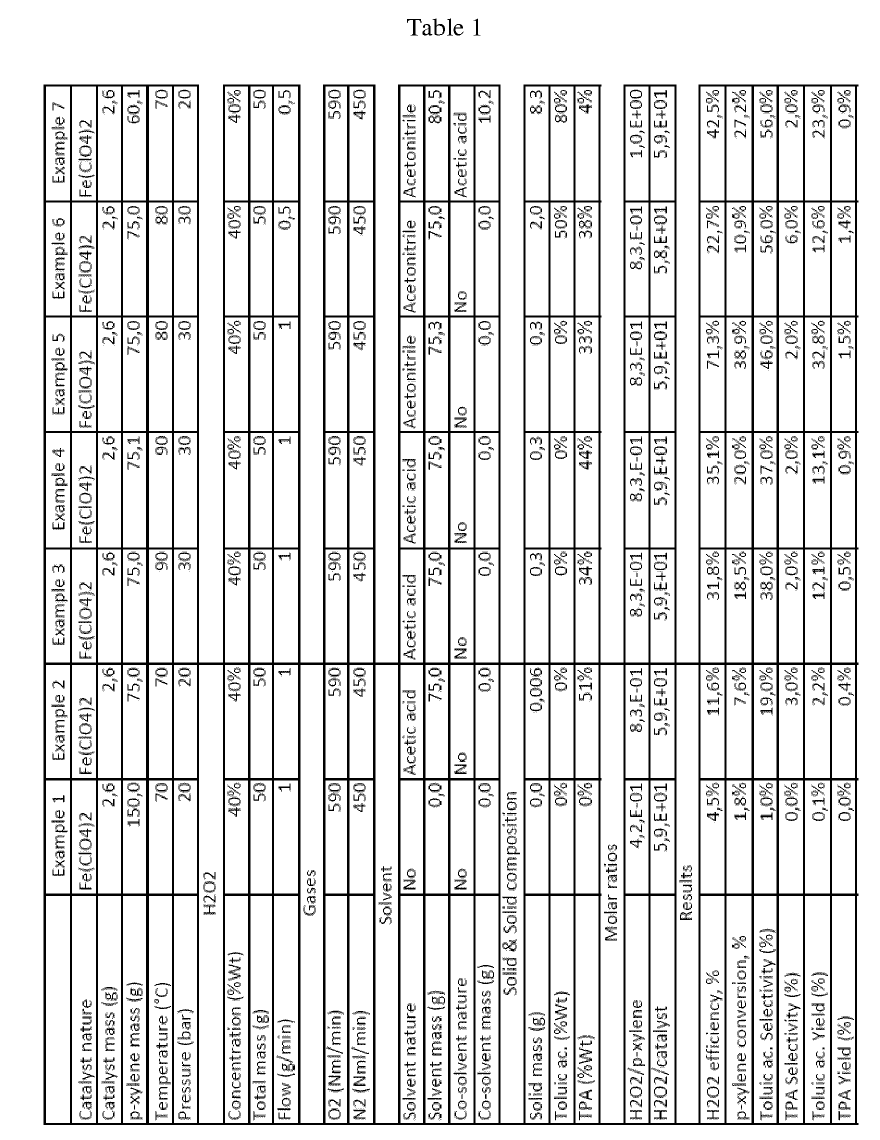

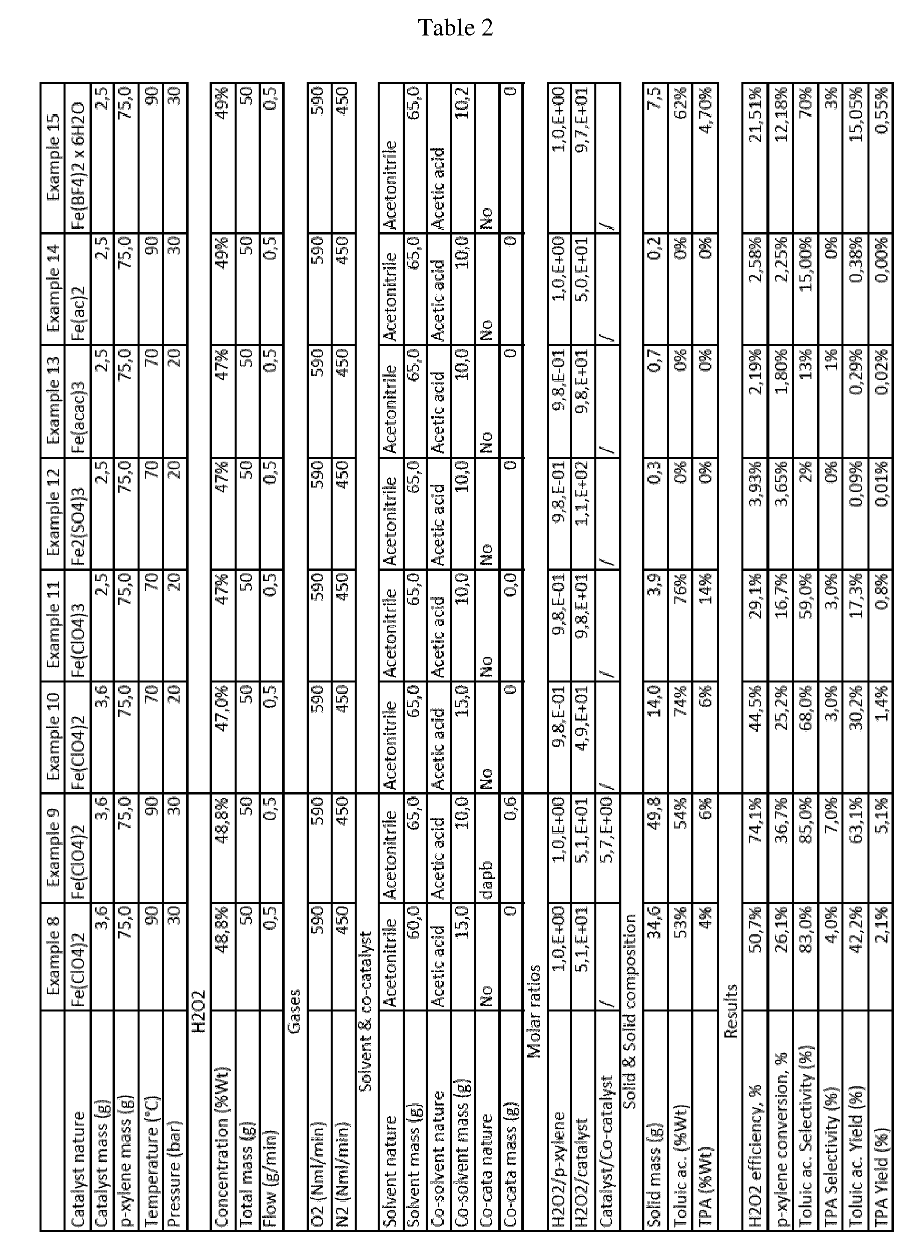

**Table 3**

| | Example 16 | Example 17 | Example 18 | Example 19 |
|---|---|---|---|---|
| Catalyst nature | Fe(ClO4)2 | Fe(ClO4)2 | Fe(ClO4)2 | Fe(ClO4)2 |
| Catalyst mass (g) | 2,6 | 2,6 | 2,6 | 2,6 |
| p-xylene mass (g) | 75,0 | 75,0 | 75,0 | 75,0 |
| Temperature (°C) | 90 | 90 | 80 | 80 |
| Pressure (bar) | 30 | 30 | 30 | 30 |

| H2O2 | | | | |
|---|---|---|---|---|
| Concentration (%Wt) | 40% | 49% | 47% | 47% |
| Total mass (g) | 50 | 50 | 50 | 50 |
| Flow (g/min) | 0,5 | 0,5 | 0,5 | 0,5 |

| Gases | | | | |
|---|---|---|---|---|
| 02 (Nml/min) | 590 | 590 | 590 | 590 |
| N2 (Nml/min) | 450 | 450 | 450 | 450 |

| Solvent & co-catalyst | | | | |
|---|---|---|---|---|
| Solvent nature | Acetonitrile | Acetonitrile | Acetonitrile | Acetonitrile |
| Solvent mass (g) | 75,0 | 75,0 | 75,0 | 75,0 |
| Co-solvent nature | No | No | No | No |
| Co-solvent mass (g) | 0,0 | 0,0 | 0,0 | 0,0 |
| Co-cata nature | No | No | No | No |
| Co-cata mass (g) | 0,0 | 0,0 | 0,0 | 0,0 |

| Molar ratios | | | | |
|---|---|---|---|---|
| H2O2/p-xylene | 8,3,E-01 | 1,0,E+00 | 9,8,E-01 | 9,8,E-01 |
| H2O2/catalyst | 5,8,E+01 | 7,0,E+01 | 6,8,E+01 | 6,8,E+01 |

| Solid & Solid composition | | | | |
|---|---|---|---|---|
| Solid mass (g) | 8,2 | 39,6 | 9,0 | 3,0 |
| Toluic ac. (%Wt) | 69% | 46% | 63% | 37% |
| TPA (%Wt) | 12% | 7% | 16% | 47% |

| Results | | | | |
|---|---|---|---|---|
| H2O2 efficiency, % | 33,2% | 55,9% | 31,0% | 18,9% |
| p-xylene conversion, % | 14,4% | 27,4% | 15,6% | 10,5% |
| Toluic ac. Selectivity (%) | 74,0% | 84,0% | 71,0% | 38,0% |
| TPA Selectivity (%) | 6,0% | 8,0% | 8,0% | 12,0% |
| Toluic ac. Yield (%) | 24,6% | 47,1% | 22,0% | 7,2% |
| TPA Yield (%) | 1,9% | 4,6% | 2,4% | 2,2% |

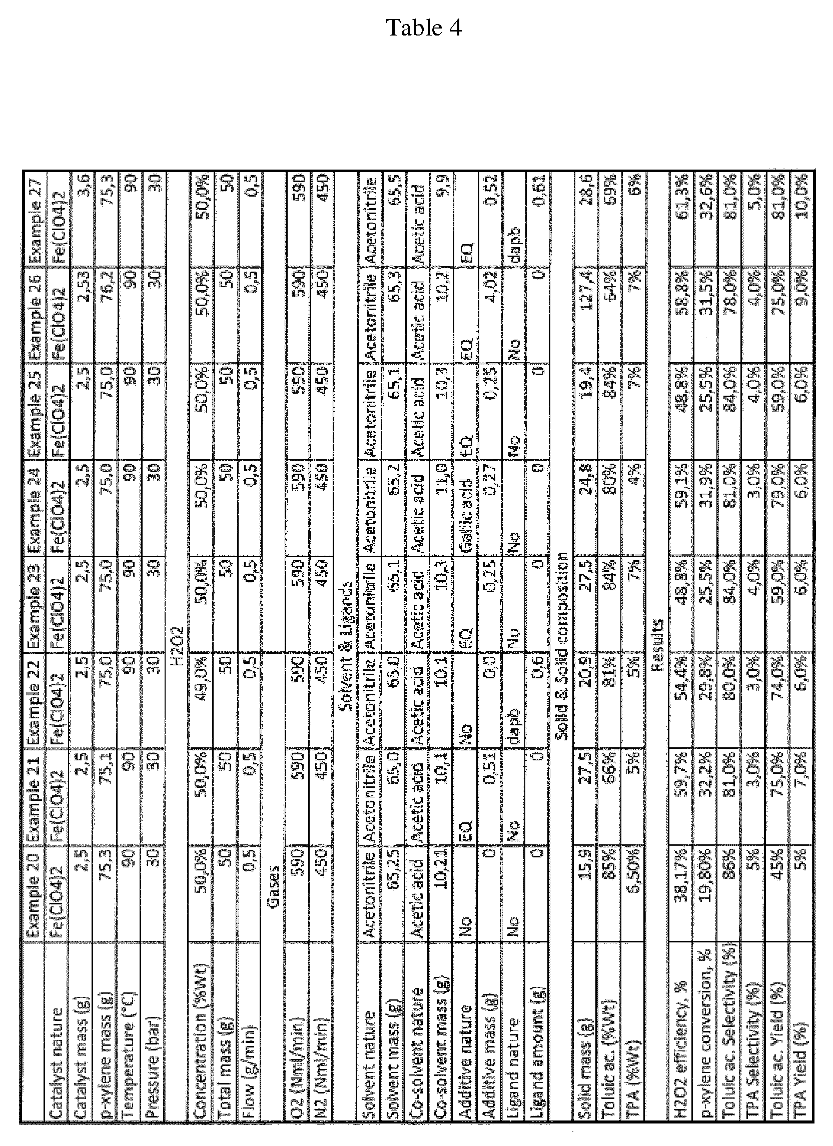

These results lead to the following comments :
Examples 1 and 2 : The use of a solvent (in this case acetic acid) enhances the reactivity of the system. Hydrogen peroxide efficiency, p-xylene conversion and the selectivities in toluic acid and in terephthalic acid (TPA) are improved. This might be explained by a better mass transfer in presence of a solvent.
Examples 2 and 3 : There is an improvement in the hydrogen peroxide efficiency, p-xylene conversion and mainly the toluic acid selectivity when raising the temperature from 70°C to 90°C. There is no effect on the TPA global yield. The pressure has been increased at higher temperature for safety reasons in order to reduce the vapor pressure of the p-xylene and so decrease the risk of gas phase explosion.
Examples 4 and 5 : The use of acetonitrile as solvent instead of acetic acid improves the hydrogen peroxide efficiency (from 20 to 38 %), the p-xylene conversion and the toluic acid selectivity (from 37 to 46 %). It is an important effect: even if the test has been done at lower temperature for safety reason (80°C instead of 90°C), the results are clearly better.
Examples 6 and 7 : In Example 7, acetic acid has been added to the reactor in a small quantity at the beginning of the reaction. Even if the test has been done at lower temperature for safety reasons (high exothermicity observed) the results are quite better than at higher temperature and without acetic acid. H2O2 efficiency has been doubled. The p-xylene conversion is three times higher. The surprising point is that the TPA yield is a little bit lower with than without acetic acid.
Examples 8 and 9 : The addition of dapb as has a positive effect on the reaction. Compared to a test done without the dapb and with a slightly higher amount of acetic acid, the H2O2 efficiency has been increased from 50 to 74 %. In presence of the dapb, the TPA selectivity and yield increase also significantly. The TPA yield is 2,1 % without dapb and 5,1 % with dapb. There is also a positive effect on the toluic acid global yield which has been increased from 42 to 63 %.
Examples 10 to 15 : Although the reaction conditions used are not quite identical, it is clear that the best results are obtained for ferrous and ferric perchlorates, followed by ferrous tetrafluoroborate. There is nearly no conversion of p-xylene in presence of Iron (III) sulfate, Iron (II) acetylacetonate and acetate. Both H2O2 efficiency and toluic acid selectivity are low with such types of catalysts which seem to be inactive for the reaction.
Examples 16 and 17 : By using a more concentrated solution of hydrogen peroxide, the reaction performances are improved : the H2O2 efficiency is doubled and the TPA yield is significantly increased (from 1,9 % to 4,6 %). Toluic acid selectivity (+10 %) and p-xylene conversion (+18 %) are also significantly enhanced.
Examples 18 and 19 : When the reaction is done in absence of oxygen, the p-xylene conversion is dropping (from 31 to 19 %). Toluic acid selectivity drops too (from 71 to 38 %) while the TPA selectivity is slightly better. However, as the conversion is lower, the global TPA yield remains the same.
Examples 3 and 16 : With a lower hydrogen peroxide introduction speed, an improvement of the p-xylene conversion and of the selectivity towards the p-toluic acid and TPA is observed.
Examples 20 to 27: These show the positive influence of the presence of Gallic Acid or EQ as additive, the latter eventually in the presence of dapb.

### 3. Tests with perchlorates of different metals

Different perchlorate based catalysts have been tested as explained above in the following conditions : 2.5 g catalyst - 10 g acetic acid - 65 g acetonitrile - 75 g p-xylene - 50 g H₂O₂ 49 %Wt (flowrate = 0.5 g/min) - 590 Nml/min Oxygen - 450 Nml/min Nitrogen - 240 minutes - 90°C - 30 bar.

The results are summarized in Table 5 below.

**Table 5 :**

| Catalyst | p-xylene Conversion (%) | p-toluic acid Selectivity (%) | TPA Selectivity (%) | H₂O₂ efficiency (%) |
|---|---|---|---|---|
| Fe(C1O₄)₃ | 16.7 | 59.0 | 3.0 | 29.1 |
| Mn(ClO₄)₂ | 2.27 | 4 | 1 | 2.5 |
| Cu(ClO₄)₂ | 1.48 | 12 | 0 | 1.7 |
| NaClO₄ | 0.10 | - | - | 0.1 |

Based on the above, the only catalysts giving good results are Iron (II) and Iron (III) perchlorate. Manganese (II) and Copper (II) perchlorate don't activate p-xylene oxidation. Sodium perchlorate is not active either.

## Claims

1. A process for oxidising an alkylaromatic compound, said process comprising a step of reacting the alkylaromatic compound with hydrogen peroxide in the presence of a catalyst comprising iron perchlorate or iron tetrafluoroborate.

2. The process according to claim 1, wherein the alkylaromatic compound comprises an aromatic ring and a (C1-C6) alkyl substituent having at least one hydrogen atom at the alpha position relative to the aromatic ring.

3. The process according to claim 2, wherein the alkylaromatic compound is a di-substituted mononuclear alkylaromatic compound.

4. The process according to claim 3, wherein the di-substituted mononuclear alkylaromatic compound is p-xylene.

5. The process according to any of the preceding claims, wherein the hydrogen peroxide is an aqueous solution having a maximum concentration between 40 and 55wt %.

6. The process according to any of the preceding claims, wherein the reaction is performed in presence of an organic solvent.

7. The process according to claim 6, wherein the organic solvent is acetonitrile or a mixture of acetonitrile and acetic acid.

8. The process according to claim 6 or 7, said process leading to a liquid organic phase, an aqueous phase and a solid phase and comprising at least one of the following steps :
1. Filtration and solid phase recovering
2. Solubilisation of the solid in an adequate solvent
3. Separation between organic and aqueous phase
4. Distillation of the organic phase for solvent recovering
5. Treatment of the aqueous phase for catalyst recovering.

9. The process according to any of the preceding claims, wherein the reaction is performed in presence of oxygen.

10. The process according to any of the preceding claims, wherein the catalyst is iron II perchlorate.

11. The process according to any of the preceding claims, wherein the iron salt catalyst is used in presence of a co-catalyst which is an organic compound.

12. The process according to the preceding claim, wherein the co-catalyst which is a Schiff base tridendate complex.

13. The process according to the preceding claim, wherein the co-catalyst is (2,6-bis-[1-(benzylimino)ethyl]pyridine - dapb).

14. An oxidation process comprising the following steps :
- a first step in which p-xylene is oxidized with hydrogen peroxide at least partly in toluic acid and terephtalic acid using a process according to any of the preceding claims;
- a second step in which the solid produced is recovered;
- a third step in which said solid is dissolved in an appropriate solvent; and
- a fourth step in which the solution is oxidized preferably using a process according to any of the preceding claims.

15. A process for making polyethylene terephthalate, said process comprising the step of polymerizing terephtalic acid obtained by a process according to the preceding claim.

16. A process according to any of the preceding claims, wherein Gallic Acid or anthraquinones are added before or during the reaction, said anthraquinones being unsubstituted or at least not substituted with hydroxyl or other oxygen containing groups.

17. The process according to the preceding claim wherein the additive is an anthraquinone substituted with aliphatic group(s), preferably ethylanthraquinone (EQ).
